# EUROPÄISCHE PATENTSCHRIFT

(11) **EP 0 755 964 B1**
(45) Veröffentlichungstag und Bekanntmachung des Hinweises auf die Patenterteilung: **23.04.2003**
(21) Anmeldenummer: 96110598.8
(22) Anmeldetag: 01.07.1996
(51) Int. Cl.: C08J 3/075, C08J 7/04, C09D 191/06, C08J 3/12

(54) **Hydrophile, hochquellfähige Hydrogele**
Hydrophylic, highly swellable hydrogels
Hydrogels hydrophyliques à pouvoir gonflant élevé

(30) Priorität: 07.07.1995 DE 19524724
(43) Veröffentlichungstag der Anmeldung: 29.01.1997
(73) Patentinhaber: BASF AKTIENGESELLSCHAFT, 67056 Ludwigshafen (DE)
(72) Erfinder: Engelhardt, Fritz, Dr., 60386 Frankfurt am Main (DE); Stüven, Uwe, Dr., 65812 Bad Soden (DE); Daniel, Thomas Dr., Chesapeake, VA 23321 (US); Herfert, Norbert, Dr., 63674 Altenstadt (DE)

(56) Entgegenhaltungen:
- EP-A- 0 096 790
- EP-A- 0 612 533
- WO-A-94/22940
- DE-A- 2 910 374
- DE-A- 4 414 117
- DATABASE WPI Section Ch, Week 8237 Derwent Publications Ltd., London, GB; Class A14, AN 82-78140E XP002040016 & JP 57 128 709 A (SUMITOMO CHEM CO LTD) , 10.August 1982

## Beschreibung

Die vorliegende Erfindung betrifft hydrophile, hochquellfähige Hydrogele, die mit nicht reaktiven, wasserunlöslichen Wachsen beschichtet sind.

Hydrophile Hydrogele, die durch Polymerisation olefinisch ungesättigter Säuren, wie beispielsweise Acrylsäure, Methacrylsäure, Acrylamidopropansulfonsäure usw., in Gegenwart geringer Mengen mehrfach olefinisch ungesättigter Verbindungen erhalten werden können, sind bereits bekannt und beispielsweise beschrieben in US 4057521, US 4062817, US 4525527, US 4286082, US 4340706 und US 4295987.

Weiterhin sind auch hydrophile Hydrogele bekannt, die durch Pfropfcopolymerisation olefinisch ungesättigter Säuren auf unterschiedliche Matrices, wie beispielsweise Polysaccharide, Polyalkylenoxide sowie deren Derivate, zugänglich sind (z.B. US 5011892, US 4076663 und US 4931497).

Die genannten Hydrogele zeichnen sich durch ein hohes Aufnahmevermögen für Wasser und wäßrige Lösungen aus und finden daher bevorzugt Anwendung als Absorptionsmittel in Hygieneartikeln.

Es ist bereits bekannt, daß die Eigenschaften dieser Hydrogele durch eine Oberflächenbehandlung mit bestimmten Substanzen modifiziert werden können. Zu diesem Zweck werden herkömmliche Hydrogele, die getrocknet, gemahlen und gegebenenfalls abgesiebt sind, in Pulverform mit reaktiven Verbindungen umgesetzt, d.h. mit Verbindungen, die mindestens zwei Gruppen enthalten, die mit den Carboxylgruppen der Hydrogele kovalente Bindungen bilden können. Es handelt sich also um eine Vernetzung, die auf der Oberfläche der Gelpartikel stattfindet.

Eine derartige Oberflächenvernetzung ist beispielsweise in der EP-A-543303 beschrieben, wobei als Oberflächenvernetzer Mischungen aus Phosphonsäurediglycidylestern und weiteren reaktiven Verbindungen eingesetzt werden.

Zahlreiche weitere Verfahren beschreiben die Oberflächenbehandlung und Vernetzung saug- und quellfähiger Polymerenpartikel mit reaktiven Verbindungen. So werden in der US 4043952 zur Verbesserung der Dispergierbarkeit in Wasser polyvalente Metallverbindungen und in der US 4051086 zur Verbesserung der Aufnahmegeschwindigkeit Glyoxal empfohlen. In den Dokumenten EP-A-83022 (zur verbesserten Dispergierbarkeit in Wasser und zur Verbesserung des Absorptionsvermögens), DE-A-3331644 (zur Verbesserung der Resistenz gegen Salzlösungen bei hoher Wasseraufnahmegeschwindigkeit), DE-A-3507775 (ebenfalls zur Erhöhung der Salzbeständigkeit bei guter Flüssigkeitsabsorption und Gelfestigkeit), DE-A-3523617 (zur Verbesserung der Fließfähigkeit und dem Verhindern des Zusammenbackens), DE-A-3628482 (zur Verbesserung der Wasseraufnahme bei wiederholter Verwendung) und EP-A-349240 (zur Erzielung eines Gleichgewichtes zwischen Absorptionsvermögen und Absorptionsgeschwindigkeit sowie Gelfestigkeit und Saugkraft) wird die Nachbehandlung von Polymeren mit zwei- oder mehrfunktionellen Gruppen enthaltenden Vernetzungsmitteln beschrieben, die mit den Carboxyl- oder Carboxylatgruppen oder anderen im Polymer enthaltenen Gruppen reagieren können. Hierbei wird entweder das Pulver direkt mit den Komponenten, ggf. unter Mitverwendung geringerer Mengen Wasser und Lösungsmittel, vermischt oder das Pulver in einem inerten Lösungsmittel dispergiert oder 10 bis 40 Gew.% Wasser enthaltende Polymere werden in einem hydrophilen oder hydrophoben Lösungsmittel dispergiert und anschließend oder gleichzeitig mit dem Vernetzungsmittel vermischt. Als Vernetzungsmittel können Polyglycidylether, Haloepoxiverbindungen, Polyole, Polyamine oder Polyisocyanate verwendet werden (siehe US 4666983). Neben diesen werden in DE-A-3314019, EP-A-317106 (jeweils zur Erreichung von hoher Absorptionsmenge und hoher Absorptionsgeschwindigkeit) und DE-A-3737196 (hohes Absorptionsvermögen und hohe Aufnahmegeschwindigkeit bei gleichzeitiger großer Gelfestigkeit) weiterhin polyfunktionelle Aziridinverbindungen, Alkyl-di- und -tri-halogenide und öllösliche Polyepoxiverbindungen erwähnt. In der DE-A-3503458 (zur Erzielung eines Polymeren mit gutem Wasserabsorptionsvermögen, hoher Wasserabsorptionsrate und hoher Gelfestigkeit bei nicht-klebrigem Gel) erfolgt die Aufbringung eines Vernetzungsmittels auf ein Polymerharz in Gegenwart eines inerten anorganischen Pulvermaterials wie SiO₂ ohne Verwendung organischer Lösungsmittel. Allen diesen Verfahren ist gemeinsam, daß anschließend eine Temperaturbehandlung der Harze erfolgt, sowie ferner, daß die zur Oberflächenbehandlung verwendeten Vernetzer wenigstens zwei funktionelle Gruppen aufweisen, d.h. reaktiv sind. DE-A-4020480 beschreibt ein Verfahren zur oberflächenvernetzung von hydrophilen Absorptionsmitteln durch Behandlung mit Alkylencarbonaten und nachfolgender thermischer Behandlung bei 150-300°C. EP-A-509708 beschreibt ein Verfahren, welches die oberflächenvernetzung carboxylgruppenhaltiger Polymerteilchen mit Polyhydroxylverbindungen in Kombination mit einer Tensidbeschichtung zum, Inhalt hat.

All diesen nach oben beschriebenen Methoden hergestellten Polymerpulvern ist gemein, daß sie einen gewissen Anteil feinerer Partikel enthalten, die für das sogenannte Stauben verantwortlich sind, und daß zum Teil diese Staubanteile infolge mechanischer Belastung, wie z.B. durch pneumatische Förderung und dadurch bedingten Abrieb, deutlich erhöht werden. Feinstäub einer Korngröße kleiner 10 µm ist aus inhalationstoxischen Gründen unerwünscht, Feinstaubanteile kleiner 100 µm verursachen das visuell sichtbare Stauben mit all seinen Folgeerscheinungen und führen zu Handlingsproblemen im Produktionsund im Verarbeitungsbetrieb und sind daher ebenfalls unerwünscht.

Desweiteren ist festzustellen, daß durch eine Nachvernetzung der Oberfläche der Gel-Partikel keine Absenkung der Hygroskopizität der getrockneten Polymerpulver erzielt wird. Diese Hygroskopizität führt zu Verbackungen der Polymerpulver an feuchter Luft und bereitet daher sowohl in den Herstellungsals auch in den Verarbeitungsbetrieben erhebliche Probleme.

Es ist bekannt, daß die Hygroskopizität dieser Polymerpulver durch Aufbringen von sehr feinteiligen anorganischen, wasserunlöslichen Pulvern auf die Oberfläche der Polymerpartikel abgesenkt werden kann. So werden in EP-A-388120 (Verringerung der Hygroskopizität, Kohäsion und Adhäsion an Metalloberflächen) Gemische aus einem hoch wasserabsorbierenden pulverigen Polymeren und einem porösen Pulver aus einem hochreinen Siliciumdioxid beschrieben, wobei das Pulver eine mittlere Teilchengröße von 0,1 bis 30 µm und eine spezifische Oberfläche von 500 m²/g oder mehr besitzt. Durch das Zumischen derart feinteiliger Pulver zu den Polymerpulvern wird jedoch die oben beschriebene Staubproblematik erheblich verschärft.

Ein weiteres Verfahren zur Oberflächenbehandlung von hydrophilen Hydrogelen dient der besseren Fixierung dieser Hydrogele an Fasermaterialien. So wird in der EP-A-612533 der Zusatz von Schmelzklebern, die bei 50 bis 200°C adhesive Eigenschaften aufweisen, zu Hydrogelen beschrieben, um für den Einsatz dieser Hydrogele in Verbindung mit Fasermaterialien die Fixierung des Hydrogels auf dem Fasermaterial zu erreichen. Als Schmelzkleber werden hier beispielsweise niedermolekulare Polyolefine, Copolymere oder Paraffinwachs verwendet. Um die gewünschte Fixierung der Hydrogele an Fasermaterialien zu erzielen, müssen diese Schmelzkleber in relativ großen Mengen zugesetzt werden. Der Zusatz von diesen relativ großen Mengen an Schmelzkleber zu den Hydrogelen führt jedoch zu einer Verminderung des Absorptionsvermögens der Hydrogele für wäßrige Flüssigkeiten, die unerwünscht ist.

DE-A-2 910 374 beschreibt fließfähige Gemische mit geringer Staubneigung aus (a) Kleinteiligen, hydrophilen Polymeren, insbesonder in Wasser löslichen oder zumindest quellbaren Polymeren, und (b) 0,01 - 10 Gew.-% einer schwerflüchtigen Komponente z.B Wachs.

Aufgabe vorliegender Erfindung ist es somit, staubfreie abriebbeständige hochquellfähige Absorptionsmittel für wäßrige Flüssigkeiten mit geringer Verbackungsneigung an feuchter Luft bereitzustellen. Diese Aufgabe wird überraschenderweise dadurch gelöst, daß an sich bekannte hydrophile, hochquellfähige Hydrogele mit nur geringen Mengen nicht reaktiver, wasserunlöslicher Wachse beschichtet werden.

Die vorliegende Erfindung betrifft somit ein hydrophiles, hochquellfähiges Hydrogel, dadurch gekennzeichnet, daß es mit einem nicht reaktiven, wasserunlöslichen Wachs beschichtet ist und die Menge an Wachs, bezogen auf die Menge an unbeschichtetem Hydrogel, von 0,05 Gew.% bis 2 Gew.% beträgt. Das Wachs kann dabei aus mehreren Komponenten bestehen.

Unter einem mit Wachs beschichteten Hydrogel ist dabei nicht zu verstehen, daß die Teilchen des Hydrogel-Pulvers an ihrer Oberfläche mit einem geschlossenen Wachsfilm bedeckt sind. Vielmehr weist die Oberfläche eines mit den erfindungsgemäßen Wachsmengen beschichteten Hydrogels Poren in ausreichender Größe und ausreichender Anzahl auf, so daß die Absorptionseigenschaften des Hydrogels nicht negativ beeinflußt werden. Wird das Hydrogel mit größeren als den erfindungsgemäßen Wachsmengen beschichtet, so nehmen Porengröße und Porenanzahl ab, und es tritt bereits eine unerwünschte Verschlechterung der Absorptionseigenschaften ein.

Unter einem Wachs ist dabei insbesondere - entsprechend der Formulierung der Deutschen Gesellschaft für Fettwissenschaft (DGF) von 1974 (s. DGF-Einheitsmethoden: Untersuchung von Fetten, Fettprodukten und verwandten Stoffen, Abteilung M: Wachse und Wachsprodukte; Wissenschaftliche Verlagsgesellschaft, Stuttgart, 1975) - eine Substanz zur verstehen, die unabhängig von ihrer chemischen Zusammensetzung und ihrer natürlichen oder künstlichen Herkunft in der Regel durch folgende mechanisch-physikalische Eigenschaften gekennzeichnet ist:
(1) bei 20°C knetbar, fest bis brüchig hart;
(2) grob- bis feinkristallin, durchscheinend bis opak, jedoch nicht glasartig;
(3) über 40°C ohne Zersetzung schmelzend;
(4) schon wenig oberhalb des Schmelzpunktes verhältnismäßig niedrigviskos und nicht fadenziehend;
(5) stark temperaturabhängig in Konsistenz und Löslichkeit;
(6) unter leichtem Druck polierbar.

Bevorzugte Wachse sind insbesondere solche, deren Schmelz- bzw. Tropfpunkte im Temperaturbereich zwischen 30 und 180°C, besonders bevorzugt zwischen 40 und 180°C, ganz besonders bevorzugt zwischen 40 und 170°C, liegen. Die Bestimmung des Tropfpunktes erfolgt dabei nach der DGF-Einheitsmethode DGF-M-III 3 (75) (Wissenschaftliche Verlagsgesellschaft, Stuttgart).

Bevorzugte Wachse sind außerdem insbesondere solche, deren Filme im Temperaturbereich zwischen O°C und 80°C nicht zum Verkleben neigen.

Die erfindungsgemäß einzusetzenden Wachse sind nicht reaktiv. Dies bedeutet im Rahmen vorliegender Erfindung, daß sie keine reaktiven Gruppen aufweisen, die mit den Carboxylgruppen an der Oberfläche der Hydrogelteilchen reagieren können.

Erfindungsgemäß einzusetzende Wachse sind z.B. Naturwachse, modifizierte Naturwachse, teilsynthetische Wachse und vollsynthetische Wachse. Beispiele für Naturwachse sind rezente Wachse, wie Pflanzenwachse oder Tierwachse. Beispiele für Pflanzenwachse sind Carnaubawachs, Candelillawachs, Ouricuriwachs, Zuckerrohrwachs und Retamowachs. Beispiel für Tierwachse sind Insektenwachse, wie Bienenwachs, Gheddawachs und Schellackwachs, sowie Wollwachs. Weitere Beispiele für Naturwachse sind fossile Wachse, wie Erdölwachse oder Braunkohlen- und Torfwachse. Beispiele für Erdölwachse sind Ozokerit und Tankbodenwachs, ein Beispiel für ein Braunkohlen- und Torfwachs ist Rohmontanwachs. Beispiele für modifizierte Naturwachse sind die durch Raffination erhaltenen Wachse, wie z.B. die aus Erdöldestillaten bzw. Destillatrückständen gewonnenen makro- und mikrokristallinen Paraffinwachse oder chemisch veränderte Wachse, wie z.B. doppelt gebleichtes Rohmontanwachs. Beispiele für teilsynthetische Wachse sind die aus Montanwachs herstellbaren Säurewachse und Esterwachse, die durch Paraffin-Oxidation herstellbaren Wachssäuren sowie Alkoholwachse und Amidwachse. Beispiele für vollsynthetische Wachse sind Kohlenwasserstoff-Wachse, wie Polyolefinwachse und Fischer-Tropsch-Wachse, sowie Synthesewachse mit sauerstoffunktionellen Gruppen. Beispiele für Synthesewachse mit sauerstoffunktionellen Gruppen sind Säurewachse, die durch Oxidation von synthetisch hergestellten Kohlenwasserstoff-Wachsen oder durch Copolymerisation bzw. Telomerisation von Olefinen mit ungesättigten Carbonsäuren entstehen, Esterwachse, die durch Veresterung synthetischer Wachssäuren mit synthetischen Alkoholen und durch Copolymerisation von Olefinen mit ungesättigten Estern, wie z.B. Vinylacetat, erhalten werden, Alkoholwachse, die durch Oxosynthese mit anschließender Hydrierung sowie durch Hydrierung synthetischer Fettsäuren hergestellt werden, sowie Amidwachse, die durch Umsetzung synthetischer Säuren mit Aminen erhalten werden. Beispiele für Wachse, die durch Oxidation von synthetisch hergestellten Kohlenwasserstoff-Wachsen erhalten werden, sind Oxidate von Polyethylenwachsen.

Bevorzugte erfindungsgemäß einzusetzende Wachse sind veredelte (d.h. entharzte und gebleichte) Montanwachse sowie Polyolefinwachse.

Besonders bevorzugte erfindungsgemäß einzusetzende Wachse sind Polyolefinwachse, wie Polyethylenwachse (Hochdruck-Polyethylenwachse, Niederdruck-Polyethylenwachse, Abbau-Polyethylenwachse), Oxidate dieser Polyethylenwachse, Wachse basierend auf Ethen-α-Olefin-Copolymeren, Wachse basierend auf Ethen-Vinylacetat-Copolymeren, Wachse, basierend auf Ethen-Styrol-Copolymeren, Wachse, basierend auf Ethen-Acrylsäure-Copolymeren, sowie Wachse, basierend auf Wachsmischungen von Polyethylenwachsen mit Poly(tetrafluorethylen)wachsen.

Es können auch Mischungen zweier oder mehrerer der oben genannten Wachse eingesetzt werden. Die Mischungsverhältnisse sind dabei vollkommen unkritisch und den jeweiligen Gegebenheiten anzupassen.

Bevorzugt ist das Hydrogel mit Wachs in einer Menge von 0,05 bis 1 Gew.%, besonders bevorzugt in einer Menge von 0,05 bis 0,95 Gew.%, ganz besonders bevorzugt in einer Menge bis 0,1 Gew.% und 0,95 Gew.%, darüber hinaus bevorzugt in einer Menge von 0,2 Gew.% bis 0,8 Gew.%, jeweils bezogen auf unbeschichtetes Hydrogel, beschichtet.

Die den erfindungsgemäßen Hydrogelen zugrunde liegenden hydrophilen, hochquellfähigen Hydrogele sind insbesondere Polymere aus (co)polymerisierten hydrophilen Monomeren, Pfropf(co)polymere von einem oder mehreren hydrophilen Monomeren auf eine geeignete Pfropfgrundlage, vernetzte Cellulose- oder Stärkeether oder in wäßrigen Flüssigkeiten quellbare Naturprodukte, wie beispielsweise Guarderivate. Diese Hydrogele sind bekannt und beispielsweise in den oben zitierten Literaturstellen beschrieben. Geeignete hydrophile Monomere sind beispielsweise polymerisationsfähige Säuren, wie Acrylsäure, Methacrylsäure, Vinylsulfonsäure, Vinylphosphonsäure, Maleinsäure einschließlich dessen Anhydrid, Fumarsäure, Itaconsäure, 2-Acrylamido-2-methylpropansulfonsäure; 2-Acrylamido-2methylpropanphosphonsäure sowie deren Amide, Hydroxyalkylester und aminogruppen- oder ammoniumgruppenhaltige Ester und Amide, desweiteren wasserlösliche N-Vinylamide oder auch Diallyldimethylammoniumchlorid.

Bevorzugte hydrophile Monomere sind Verbindungen der allgemeinen Formel I, 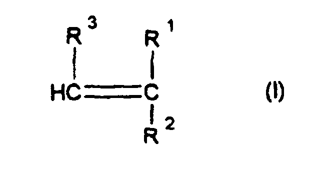 worin
- R¹: Wasserstoff, Methyl oder Ethyl,
- R²: die Gruppe -COR⁴, die Sulfonsäuregruppe, die Phosphonsäuregruppe, die mit (C₁-C₄)-Alkanol veresterte Phosphonsäuregruppe oder eine Gruppe der Formel 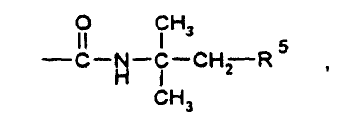
- R³: Wasserstoff, Methyl, Ethyl oder die Carboxylgruppe,
- R⁴: Hydroxy, Amino oder Hydroxy-(C₁-C₄)-alkyl und
- R⁵: die Sulfonsäuregruppe, die Phosphonsäuregruppe, die mit (C₁-C₄)-Alkanol veresterte Phosphonsäuregruppe oder die Carboxylgruppe bedeuten.

Beispiele für (C₁-C₄)-Alkanole sind Methanol, Ethanol, n-Propanol, i-Propanol oder n-Butanol.

Besonders bevorzugte hydrophile Monomere sind Acrylsäure und Methacrylsäure.

Geeignete Pfropfgrundlagen können natürlichen oder synthetischen Ursprungs sein. Beispiele sind Stärke, Cellulose oder Cellulosederivate sowie andere Polysaccharide und Oligosaccharide, Polyalkylenoxide, insbesondere Polyethylenoxide und Polypropylenoxide, sowie hydrophile Polyester.

Geeignete Polyalkylenoxide haben beispielsweise die Formel 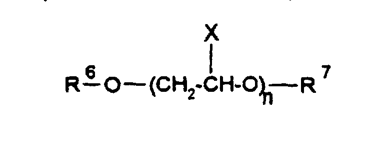 worin R⁶ und R⁷ unabhängig voneinander Wasserstoff, Alkyl, Alkenyl oder Aryl,
- X: Wasserstoff oder Methyl und
- n: eine ganze Zahl von 1 bis 10000 bedeuten.

R⁶ und R⁷ bedeuten bevorzugt Wasserstoff, (C₁-C₆)-Alkyl, (C₂-C₆)-Alkenyl oder Phenyl.

Beispiele für (C₁-C₆)-Alkyl sind Methyl, Ethyl oder n-Butyl, für (C₂-C₆)-Alkenyl Vinyl oder Allyl.

Bevorzugte Hydrogele sind insbesondere Polyacrylate, Polymethacryalate sowie die in US 4931497, US 5011892 und US 5041496 beschriebenen Pfropfpolymere.

Die den erfindungsgemäßen Hydrogelen zugrunde liegenden hydrophilen, hochquellfähigen Hydrogele sind bevorzugt vernetzt, d.h. sie enthalten Verbindungen mit mindestens zwei Doppelbindungen, die in das Polymernetzwerk einpolymerisiert sind.

Geeignete Vernetzer sind insbesondere Methylenbisacryl- bzw. -methacrylamid, Ester ungesättigter Mono- oder Polycarbonsäuren von Polyolen, wie Diacrylate oder Triacrylate, z.B. Butandiol- oder Ethylenglykoldiacrylat bzw. -methacrylat sowie Trimethylolpropantriacrylat, und Allylverbindungen wie Allyl(meth)acrylat, Triallylcyanurat, Maleinsäurediallylester, Polyallylester, Tetraallyloxyethan, Triallylamin, Tetraallylethylendiamin, Allylester der Phosphorsäure sowie Vinylphosphonsäurederivate, wie sie beispielsweise in der EP-A-343427 beschrieben sind.

Darüber hinaus sind die den erfindungsgemäßen Hydrogelen zugrunde liegenden hydrophilen, hochquellfähigen Hydrogele besonders bevorzugt in an sich bekannter Weise in wäßriger Gelphase nachvernetzt oder als gemahlene und abgesiebte Polymerpartikel oberflächenvernetzt. Dazu geeignete Vernetzer sind Verbindungen, die mindestens zwei Gruppen enthalten, die mit den Carboxylgruppen des hydrophilen Polymeren kovalente Bindungen bilden können. Geeignete Verbindungen sind beispielsweise Di- oder Polyglycidylverbindungen, wie Phosphonsäurediglycidylester oder Glycidylether von Di- oder Polyolen, Alkoxysilylverbindungen, Polyaziridine, Polyamine oder Polyamidoamine, wobei die genannten Verbindungen auch in Mischungen untereinander verwendet werden können (siehe beispielsweise EP-A-83022, EP-A-543303 und EP-A-530438). Als Vernetzer geeignete Polyamidoamine sind insbesondere in der EP-A-349935 beschrieben.

Die den erfindungsgemäßen Hydrogelen zugrunde liegenden hydrophilen, hochquellfähigen Hydrogele können durch bekannte Polymerisationsverfahren hergestellt werden. Bevorzugt ist die Polymerisation in wäßriger Lösung nach dem Verfahren der sogenannten Gelpolymerisation. Dabei werden 15- bis 50gew.%ige wäßrige Lösungen eines oder mehrerer hydrophiler Monomerer und gegebenenfalls einer geeigneten Pfropfgrundlage in Gegenwart eines Radikalinitiators bevorzugt ohne mechanische Durchmischung unter Ausnutzung des Trommsdorff-Norrish-Effektes (Bios Final Rep. 363.22; Makromol. Chem. 1, 169 (1947)), polymerisiert.

Die Polymerisationsreaktion kann im Temperaturbereich zwischen O°C und 130°C, vorzugsweise zwischen 10°C und 100°C, sowohl bei Normaldruck als auch unter erhöhtem Druck durchgeführt werden. Wie üblich kann die Polymerisation auch in einer Schutzgasatmosphäre, vorzugsweise unter Stickstoff, ausgeführt werden.

Die erfindungsgemäßen Hydrogele können dadurch hergestellt werden, daß die nicht reaktiven, wasserunlöslichen Wachse in an sich bekannter Weise im gewünschten Gewichtsverhältnis auf das in der Regel durch Zerkleinerung, Trocknung, Mahlung und Siebung des Polymers erhaltene Pulver der zugrunde liegenden hydrophilen, hochquellfähigen Hydrogele aufgebracht und Temperaturen oberhalb des Schmelz- oder Tropfpunktes der Wachse ausgesetzt werden. Dieses Aufbringen geschieht bevorzugt in Mischern, wie beispielsweise Zwillingstrommelmischern, sogenannten "ZIG-ZAG"-Mischern, horizontal arbeitenden Pflugscharmischern, wie z.B. Lödige-Mischern oder Kegel-Schneckerimischern oder senkrecht zylindrischen Mischern mit koaxial rotierenden Messern oder auch Wirbelschichtmischern. Die Temperaturbehandlung kann gleichzeitig zum Aufbringen in einem üblichen, oben beschriebenen heizbaren Mischaggregat erfolgen. In einer bevorzugten Ausführungsform erfolgt der Temperaturbehandlungsschritt jedoch in einem nachgeschalteten Trockner über einen Zeitraum von 5 Minuten bis 6 Stunden. Die Temperaturbehandlung erfolgt - je nach Schmelz- bzw. Tropfpunkt der einzusetzenden Wachse - bei 30 bis 200°C.

Die nicht reaktiven, wasserunlöslichen Wachse können dabei in Pulverform auf die hydrophilen, hochquellfähigen Hydrogel-Pulver aufgebracht werden.

Bevorzugt werden die nicht reaktiven, wasserunlöslichen Wachse jedoch in Form einer wäßrigen Wachsdispersion, Wachsemulsion oder Wachssuspension eingesetzt. Sie können aber auch in Form einer Lösung in einem organischen Lösungsmittel oder in einem Gemisch aus Wasser und einem organischen wassermischbaren Lösungsmittel eingesetzt werden. Auch die genannten wäßrigen Dispersionen, Emulsionen und Suspensionen können einen Anteil an organischem, gegebenenfalls wassermischbaren Lösungsmittel enthalten.

Geeignete organische Lösungsmittel sind beispielsweise aliphatische und aromatische Kohlenwasserstoffe, Alkohole, Ether, Ester und Ketone wie beispielsweise n-Hexan, Cyclohexan, Toluol, Xylol, Methanol, Ethanol, i-Propanol, Ethylenglykol, 1,2-Propandiol, Glycerin, Diethylether, Methyltriglykol, Polyethylenglykole mit mittlerem Molekulargewicht von 200 - 10000, Ethylacetat, n-Butylacetat, Aceton und 2-Butanon.

Geeignete wassermischbare organische Lösungsmittel sind beispielsweise aliphatische (C₁-C₄)-Alkohole wie beispielsweise Methanol, i-Propanol, t-Butanol, mehrwertige Alkohole wie beispielsweise Ethylenglykol, 1,2-Propandiol und Glycerin, Ether wie beispielsweise Methyltriglykol und Polyethylenglykole mit mittlerem Molekulargewicht von 200 - 10000 sowie Ketone wie beispielsweise Aceton und 2-Butanon.

Im Falle von oberflächennachvernetzten, hydrophilen hochquellfähigen Hydrogelen kann das Aufbringen der nicht reaktiven, wasserunlöslichen Wachse vor dem Oberflächennachvernetzungsschritt, während des Oberftächennachvernetzungsschrittes oder nach dem Oberflächennachvernetzungsschritt erfolgen.

Gegenstand der vorliegenden Erfindung ist auch ein Verfahren zur Verringerung des Staubens eines hydrophilen, hochquellfähigen Hydrogels, dadurch gekennzeichnet, daß das Hydrogel mit einem Wachs in einer Menge von 0,05 Gew.% bis 2 Gew.%, bezogen auf das unbeschichtete Hydrogel, beschichtet wird. Hinsichtlich der Hydrogele, der Wachse, der Aufbringung des Wachses auf das Hydrogel und der bevorzugten Ausführungsformen gilt das oben Gesagte entsprechend.

Die erfindungsgemäßen Hydrogele zeichnen sich durch eine hervorragende mechanische Stabilität, insbesondere Abriebfestigkeit, aus. Dies gilt besonders bei der Einarbeitung in Hygieneartikel. Außerdem weisen sie lediglich eine minimale Staubentwicklung auf und zeigen keine Neigung zu Verbackungen in feuchter Luft.

Sie sind deshalb in hervorragender Weise als Absorptionsmittel für Wasser und wäßrige Flüssigkeiten, insbesondere von Körperflüssigkeiten, wie z.B. Urin oder Blut, geeignet, beispielsweise in Hygieneartikeln wie z.B. Baby- , und Erwachsenenwindeln, Damenbinden, Tampons und dergleichen. Sie können aber auch als Bodenverbesserungsmittel in Landwirtschaft und Gartenbau, als Feuchtigkeitsbindemittel bei der Kabelummantelung sowie zum Eindicken wäßriger Abfälle verwendet werden.

Von den in den nachfolgenden Beispielen beschriebenen erfindungsgemäßen Hydrogelen wurde die Abriebfestigkeit und das Anti-caking-Verhalten bestimmt.

Die Bestimmung der Abriebfestigkeit erfolgte in einer zylindrischen Porzellanmühle eines Innendurchmessers von 11 cm, einer inneren Höhe von 11,5 cm, einem Fassungsvolumen von ca. 1300 ml und dazugehörigen Metallkugeln (32 Stück mit einem Durchmesser von je ca. 0,9 cm und einem Gewicht von je ca. 16,5 g sowie 1 Stück mit einem Durchmesser von ca. 6,4 cm und einem Gewicht von ca. 1324 g) mit einem Gesamtgewicht von ca. 1852 g. Das Gefäß wurde mit den Kugeln sowie jeweils 100 g des zu prüfenden Polymerpulvers befüllt, verschlossen und für 30 Minuten bei 60 UpM auf entsprechendem Walzenantrieb gerollt. Das Polymerpulver wurde vor und nach dieser Behandlung einer Siebanalyse unterzogen, wobei insbesondere der Anteil im unteren Kornbereich und die Absorption unter Druck (AUL) bei unterschiedlicher Druckbelastung und Flächenbelegung bestimmt wurden.

Die Absorption unter Druck wurde in bekannter Weise, wie beispielsweise in der EP-A-339 461 beschrieben, bestimmt.

Zur Bestimmung des Anti-caking-Verhaltens wurden 5,0 g des zu prüfenden Polymerpulvers in ein Becherglas eingewogen und für 30 Minuten in einem Klimaschrank einer Temperatur von 40°C und einer relativen Luftfeuchtigkeit von 95 % ausgesetzt. Danach erfolgte eine visuelle Beurteilung des Anti-caking-Verhaltens, wobei die Beurteilungsskala von 1 (klumpenfreies, frei fließendes Polymerpulver) bis 5 (völlig zusammengebackenes Polymerpulver) reicht.

### Beispiel 1:

In einem Telschig-Laborsprühmischer RSM 6-60 von 6 l Inhalt wurden 1 kg Superabsorber, hergestellt analog Beispiel 5 der DE-A-4138408, deren Inhalt ausdrücklich Bestandteil der vorliegenden Offenbarung ist, vorgelegt. Unter Mischen wurde auf dieses bereits oberflächenvernetztes *Superabsorber*-Granulat im Verlauf von 10 Minuten mit Hilfe einer Zweistoffdüse eine Dispersion aus 4 g LANCO PE W 1555 und 30 g Wasser aufgesprüht und 3 Minuten nachgemischt. Das Produkt wurde anschließend im Trockenschrank 30 Minuten bei 140°C nachgetrocknet. Eventuell gebildete Klumpen wurden durch Absieben über ein Sieb mit 0,85 mm Maschenweite entfernt. Von dieser Ware wurden die Abriebfestigkeit sowie das Anti-caking-Verhalten wie oben beschrieben bestimmt. Die Absorption unter Druck wurde bei einer Druckbelastung von 40 g/cm² und einer Flächenbelegung von 0,032 g/cm² ermittelt, wobei die Kornfraktion 0,3 bis 0,6 mm verwendet wurde.

Der Versuch wurde mit drei weiteren Wachs-Typen wiederholt. Die Ergebnisse sind Tabelle 1 zu entnehmen.

**Tabelle 1**

| | | vor Abriebtest | | nach Abriebtest | |
|---|---|---|---|---|---|
| Wachs-Typ | Anti-caking | AUL (g/g) | Komanteil < 200 µm | AUL (g/g) | Kornanteil < 200 µm |
| ohne Nachbehandlung | 4-5 | 23,0 | 10,5 % | 15,9 | 27,9 % |
| | | | | | |
| LANCO PE W 1555 ^{a)} | 1-2 | 29,2 | 10,0 % | 27,3 | 16,0 % |
| | | | | | |
| LANCO TFW 1765^{b)} | 1 | 29,1 | 8,3 % | 26,2 | 16,5 % |
| | | | | | |
| Hoechst-Wachs DPE^{c)} | 2 | 27,5 | 9,8 % | 24,3 | 18,8 % |
| | | | | | |
| Hoechst Wachs PED 522^{d)} | 1-2 | 28,5 | 10,4 % | 27,0 | 16,9 % |

| | | | | | |
|---|---|---|---|---|---|
| a) LANCO PE W 1555 ist ein mikronisiertes Polyethylen-Wachs der Firma Langer & Co. | | | | | |
| b) LANCO TF W 1765 ist ein Polytetrafluorethylen/Polyethylen-Wachs der Firma Langer & Co. | | | | | |
| c) Hoechst-Wachs DPE ist ein Esterwachs auf Basis von raffiniertem Montanwachs der Firma Hoechst AG. | | | | | |
| d) Hoechst-Wachs PED 522 ist ein polares Polyethylen-Wachs der Firma Hoechst AG. | | | | | |

### Beispiel 2

In einem Lödige-Labormischer M5R wurden 1 kg Superabsorber, hergestellt analog Beispiel 5a der DE-A-4138408, vorgelegt. Unter Mischen wurden auf dieses noch nicht oberflächennachvernetzte Superabsorber-Granulat im Verlauf von 5 Minuten mit Hilfe einer Zweistoffdüse 100 g einer Vernetzer-Lösung, bestehend aus 2,5 Gew.% n-Propylphosphonsäurediglycidylester, 2,5 Gew.-% Monoethylenglykoldiglycidylether (epoxy equivalent g/equ. = 112), 47,5 Gew.% i-Propanol und 47,5 Gew.% Wasser, aufgesprüht und 2 Minuten nachgemischt. Anschließend wurde auf dieses feuchte Superabsorber-Granulat innerhalb von 5 Minuten mit Hilfe einer Zweistoff-Düse eine Dispersion aus 4 g Hoechst-Wachs PED 522 und 30 g Wasser aufgesprüht und 2 Minuten nachgemischt. Das Produkt wurde danach im Trockenschrank 60 Minuten bei 140°C behandelt. Eventuell gebildete Klumpen wurden durch Absieben über ein Sieb mit 0,85 mm Maschenweite entfernt.

Von dieser Ware wurde die Abriebfestigkeit und das Anticaking-Verhalten wie oben beschrieben bestimmt. Außerdem erfolgte eine Bestimmung der Staubanteile (Partikel <10 gm) über eine Laser-Partikelgrößenanalyse nach dem Trockendispergierverfahren nach vorheriger Heraussiebung der Partikel mit einer Korngröße von größer 250 µm, d.h. Absiebung über ein Sieb mit einer Maschenweite von 250 µm. Diese Bestimmungsmethode eignet sich allerdings nur für Relativmessungen zu Vergleichszwecken, da sie, bedingt durch die Meßmethodik, zu hohe Werte für die unteren Partikelgrößen angibt.

Der Versuch wurde mit drei weiteren Wachs-Typen wiederholt. Bei dem Kontroll-Versuch wurde nur VernetzerLösung, aber keine Wachs-Dispersion, aufgesprüht. Die Ergebnisse sind Tabelle 2 zu entnehmen.

**Tabelle 2**

| | | Kornanteil < 200 µm | | |
|---|---|---|---|---|
| Wachs-Typ | Anti-caking | Feinstaub-anteil¹⁾ | vor Abriebtest | nach Abriebtest |
| Kontrollversuch | 4-5 | 0.089 % | 6,5 % | 17,9 % |
| | | | | |
| Hoechst-Wachs PED 522 ^{d)} | 1-2 | 0.032 % | 5,8 % | 10,1 % |
| | | | | |
| Hoechst-Wachs DPE ^{c)} | 2 | 0,042 % | 5,2 % | 9,8 % |
| | | | | |
| Hoechst-Wachs S ^{e)} | 2-3 | 0,047 % | 6,2 % | 10,7 % |
| | | | | |
| LANCO Pe W 1555^{a)} | 1 | 0,024 % | 4,9 % | 8,1 % |

| | | | | |
|---|---|---|---|---|
| 1) Anteil des Feinstaubs mit einer Korngröße kleiner 10 µm im Superabsorber-Pulver, angegeben in Gewichtsprozent | | | | |
| a) LANCO PE W 1555 ist ein mikronisiertes Polyethylen-Wachs der Firma Langer & Co. | | | | |
| c) Hoechst-Wachs DPE ist ein Esterwachs auf Basis von raffiniertem Montanwachs der Firma Hoechst AG. | | | | |
| d) Hoechst-Wachs PED 522 ist ein polares Polyethylenwachs der Firma Hoechst AG. | | | | |
| e) Hoechst-Wachs S ist ein Säurewachs auf Basis von raffiniertem Montanwachs der Firma Hoechst AG. | | | | |

### Beispiel 3

In einem Telschig-Laborsprühmischer RSM 6-60 von 6 1 Inhalt wurden 1 kg Superabsorber, hergestellt analog Beispiel 5a) der DE-A-4138408, und 4 g LANCO PE W 1555Wachspulver innerhalb von 20 Minuten innig vermischt. Dieses Gemisch wurde anschließend im Trockenschrank 45 Minuten bei 140°C getempert. Unter erneutem Mischen im Telschig-Laborsprühmischer RSM 6-60 wurden auf dieses Superabsorber-Granulat im Verlauf von 10 Minuten mit Hilfe einer Zweistoffdüse 100 g einer Vernetzer-Lösung, bestehend aus 2,5 Gew. % n-Propylphosphonsäurediglycidylester, 2,5 Gew.% Monoethylenglykoldiglycidylether (epoxy equivalent g/equ. = 112), 47,5 Gew.% i-Propanol und 47,5 Gew.% Wasser, aufgesprüht und 5 Minuten nachgemischt. Das Produkt wurde danach im Trockenschrank 60 Minuten bei 140°C behandelt. Eventuell gebildete Klumpen wurden durch Absieben über ein Sieb mit 0,85 mm Maschenweite entfernt.

Von dieser Ware wurde die Abriebfestigkeit sowie das Anti-caking-Verhalten wie oben beschrieben bestimmt. Die Absorption unter Druck wurde bei einer Druckbelastung von 40 g/cm² und einer Flächenbelegung von 0,032 g/cm² ermittelt, wobei die Kornfraktion 0,3 bis 0,6 mm verwendet wurde.

Der Versuch wurde mit zwei weiteren Wachs-Typen wiederholt. Bei dem Kontroll-Versuch wurde die Vernetzer-Lösung direkt auf den nach Beispiel 5a) der DE-A-4138408 hergestellten Superabsorber aufgesprüht, ohne vorheriges Mischen mit Wachs-Pulver. Die Ergebnisse sind Tabelle 3 zu entnehmen.

**Tabelle 3**

| | vor Abriebtest | | | nach Abriebtest | |
|---|---|---|---|---|---|
| Wachs-Typ | Anti-caking | AUL (g/g) | Kornanteil < 200 µm | AUL (g/g) | Kornanteil < 200 µm |
| Kontrollversuch | 4-5 | 2405 | 6,8 | 18,5 | 18,5 % |
| LANCO PE W 1555 ^{a)} | 1-2 | 29,7 | 5,7 % | 26,8 | 8,4 % |
| Hoechst-Wachs PED 136 ^{f)} | 1-2 | 28,0 | 5,5 % | 26,3 | 8,9 % |
| Hoechst Wachs UL^{g)} | 2 | 26.9 | 6,2 % | 25,8 | 10,3 % |

| | | | | | |
|---|---|---|---|---|---|
| a) LANCO PE W 1555 ist Wachs der Firma Langer ein mikronisiertes Polyethylen& Co. | | | | | |
| f) Hoechst-Wachs PED 136 ist ein polares Polyethyten-Wachs der Firma Hoechst AG. | | | | | |
| g) Hoechst-Wachs UL ist ein Säurewachs auf Basis von raffiniertem Montanwachs der Firma Hoechst AG. | | | | | |

## Patentansprüche

1. Hydrophiles, hochquellfähiges Hydrogel, **dadurch gekennzeichnet, daß** es mit einem nicht reaktiven, wasserunlöslichen Wachs beschichtet ist und die Menge an Wachs, bezogen auf die Menge an unbeschichtetem Hydrogel, von 0,05 bis 2 Gew.% beträgt.

2. Hydrogel nach Anspruch 1, **dadurch gekennzeichnet, daß** das Wachs einen Schmelz- bzw. Tropfpunkt im Temperaturbereich zwischen 30 und 180°C aufweist.

3. Hydrogel nach Anspruch 1 und/oder 2, **dadurch gekennzeichnet, daß** das Wachs ein vereiteltes Montanwachs ist.

4. Hydrogel nach Anspruch 1 und/oder 2, **dadurch gekennzeichnet, daß** das Wachs ein Polyethylenwachs oder ein Oxidat eines Polyethylenwachses ist.

5. Hydrogel nach einem oder mehreren der Ansprüche 1 bis 4, **dadurch gekennzeichnet, daß** es mit Wachs in einer Menge von 0,1 bis 0,95 Gew.%, bezogen auf das unbeschichtete Hydrogel, beschichtet ist.

6. Hydrogel nach einem oder mehreren der Ansprüche 1 bis 5, **dadurch gekennzeichnet, daß** es ein Polymer aus (co)-polymerisierten hydrophilen Monomeren, ein Pfropf(co)polymer von einem oder mehreren hydrophilen Monomeren auf eine geeignete Pfropfgrundlage, ein vernetzter Cellulose- oder Stärkeether oder ein in wäßrigen Flüssigkeiten quellbarer Naturstoff ist.

7. Verfahren zur Herstellung eines Hydrogels gemäß einem oder mehreren der Ansprüche 1 bis 6, **dadurch gekennzeichnet, daß** das nicht reaktive, wasserunlösliche Wachs auf das zugrunde liegende hydrophile, hochquellfähige Hydrogel aufgebracht wird.

8. Verfahren zur Verringerung des Staubens eines hydrophilen, hochquellfähigen Hydrogels, **dadurch gekennzeichnet, daß** das Hydrogel mit einem Wachs in einer Menge von 0,05 Gew.% bis 2 Gew.%, bezogen auf das unbeschichtete Hydrogel, beschichtet wird.

9. Verwendung der Hydrogele gemäß einem oder mehreren der Ansprüche 1 bis 6 als Absorptionsmittel für Wasser und wäßrige Flüssigkeiten.

10. Verwendung gemäß Anspruch 9 zur Absorption von Körperflüssigkeiten, insbesondere in Hygieneartikeln, wie z.B. Windeln, Tampons oder Damenbinden.

## Claims

1. A hydrophilic, highly swellable hydrogel, which is coated with an unreactive, water-insoluble wax in an amount, based on the amount of uncoated hydrogel, of from 0.05 to 2% by weight.

2. A hydrogel as claimed in claim 1, wherein the wax has a melting point or dropping point in the temperature range between 30 and 180°C.

3. A hydrogel as claimed in claim 1 and/or 2, wherein the wax is a modified montan wax.

4. A hydrogel as claimed in claim 1 and/or 2, wherein the wax is a polyethylene wax or an oxidate of a polyethylene wax.

5. A hydrogel as claimed in any of claims 1 to 4, which is coated with wax in an amount of from 0.1 to 0.95% by weight, based on the uncoated hydrogel.

6. A hydrogel as claimed in any of claims 1 to 5, which is a polymer of (co)polymerized hydrophilic monomers, a graft (co)polymer of one or more hydrophilic monomers on a suitable grafting base, a crosslinked cellulose ether or starch ether or a natural substance swellable in aqueous liquids.

7. A process for preparing a hydrogel as claimed in any of claims 1 to 6, which comprises applying the unreactive, water-insoluble wax to the parent hydrophilic, highly swellable hydrogel.

8. A process for reducing the dusting of a hydrophilic, highly swellable hydrogel, which comprises coating the hydrogel with a wax in an amount of from 0.05% by weight to 2% by weight, based on the uncoated hydrogel.

9. The use of the hydrogels as claimed in any of claims 1 to 6 as absorbents for water and aqueous liquids.

10. The use as claimed in claim 9 for absorbing body fluids, in particular in hygiene articles, for example diapers, tampons or sanitary napkins.

## Revendications

1. Hydrogel hydrophile à haut pouvoir de gonflement, **caractérisé en ce qu'**il est enrobé d'une cire non réactive et insoluble dans l'eau et que la quantité de cire, par rapport à la quantité d'hydrogel non enrobé, est de 0,05 à 2% en poids.

2. Hydrogel selon la revendication 1, **caractérisé en ce que** la cire présente un point de fusion et / ou un point de goutte situé dans une plage de température de 30 à 180°C.

3. Hydrogel selon la revendication 1 et / ou 2, **caractérisé en ce que** la cire est une cire montanique raffinée.

4. Hydrogel selon la revendication 1 et / ou 2, **caractérisé en ce que** la cire est une cire de polyéthylène ou une cire de polyéthylène oxydée.

5. Hydrogel selon l'une ou plusieurs des revendications 1 à 4, **caractérisé en ce qu'**il est enrobé de cire en une quantité de 0,1 à 0,95% en poids, par rapport à l'hydrogel non enrobé.

6. Hydrogel selon l'une ou plusieurs des revendications 1 à 5, **caractérisé en ce qu'**il est un polymère à base de monomères hydrophiles (co)polymérisés, un copolymère greffé d'un ou plusieurs monomères hydrophiles sur une base de greffage appropriée, une cellulose réticulée ou un éther d'amidon ou une matière naturelle gonflable dans des liquides aqueux.

7. Procédé de préparation d'un hydrogel selon l'une ou plusieurs des revendications 1 à 6, **caractérisé en ce qu'**une cire non réactive, insoluble dans l'eau, est appliquée sur l'hydrogel à haut pouvoir gonflant de base.

8. Procédé de réduction du peluchage d'un hydrogel hydrophile à haut pouvoir gonflant, **caractérisé en ce que** l'hydrogel est enrobé d'une cire en une quantité de 0,05% en poids à 2% en poids, par rapport à l'hydrogel non enrobé.

9. Utilisation des hydrogels selon l'une ou plusieurs des revendications 1 à 6 comme moyen d'absorption pour de l'eau et des liquides aqueux.

10. Utilisation selon la revendication 9 pour l'absorption de liquides corporels, en particulier dans des articles d'hygiène, comme par exemple des couches, des tampons ou des serviettes hygiéniques.
